# EUROPÄISCHE PATENTSCHRIFT

(11) **EP 2 729 441 B1**
(45) Veröffentlichungstag und Bekanntmachung des Hinweises auf die Patenterteilung: **23.08.2017**
(21) Anmeldenummer: 12734903.3
(22) Anmeldetag: 05.07.2012
(51) Int. Cl.: C07C 68/02, C07C 68/08, C07C 69/96

(54) **VERFAHREN ZUR HERSTELLUNG VON DIARYLCARBONATEN**
METHOD FOR MANUFACTURING DIARYL CARBONATES
PROCÉDÉ DE FABRICATION DE DIARYLCARBONATES

(30) Priorität: 08.07.2011 EP 11173302
(43) Veröffentlichungstag der Anmeldung: 14.05.2014
(73) Patentinhaber: Covestro Deutschland AG, 51373 Leverkusen (DE)
(72) Erfinder: OOMS, Pieter, 47800 Krefeld (DE); RISSE, Friedhelm, 50739 Köln (DE); DÜX, Andre, 53332 Bornheim (DE); BUCHALY, Carsten, 40233 Düsseldorf (DE); LEIBERICH, Ricarda, 63263 Neu-Isenburg (DE); RONGE, Georg, 40549 Düsseldorf (DE); KRÄMER, Korbinian, 50733 Köln (DE); SCHELLEN, Ralph, 41541 Dormagen (DE); KÖHLER, Karl-Heinz, 52078 Aachen-Brand (DE); VANDEN EYNDE, Johan, 9052 Zwijnaarde (BE)
(74) Vertreter: Levpat
(86) Internationale Anmeldenummer: PCT/EP2012/063145
(87) Internationale Veröffentlichungsnummer: WO 2013/007601

(56) Entgegenhaltungen:
- EP-A2- 1 112 997
- EP-A2- 1 234 845
- EP-B1- 2 729 440
- WO-A1-95/25083
- DE-A1- 2 447 348
- US-A- 2 362 865

## Beschreibung

Gegenstand der Erfindung, die lediglich durch den Ansprüchen definiert ist, ist ein Verfahren zur Herstellung von Diarylcarbonaten aus Monohydroxy-aromaten und Phosgen in Gegenwart definierter Mengen an ausgewählten Katalysatoren, die im flüssigen Reaktionsgemisch homogen löslich sind. Insbesondere ist es ein Verfahren zur Herstellung von Diphenylcarbonat (DPC) aus Phenol und Phosgen zumindest teilweise in flüssiger Phase ohne Verwendung zusätzlicher Lösungsmittel, wobei das hergestellte DPC einen verminderten Gehalt an Salicylsäurephenylester (Salol) aufweist und der bei der Reaktion gewonnene Chlorwasserstoff weitgehend frei von Phosgen ist. Das Verfahren wird bevorzugt bei Temperaturen oberhalb von 80°C durchgeführt, um Abscheidungen von gebildetem DPC in fester Form zu vermeiden. Die Umsetzung der Edukte kann sowohl bei Normaldruck oder leicht vermindertem Druck als auch bei erhöhten Drücken von bis zu 50 bar (a), bei denen auch das Phosgen in kondensierter Phase vorliegt, durchgeführt werden. Das nach diesem Verfahren hergestellte DPC ist aufgrund seiner hohen Reinheit besonders geeignet für die Herstellung von hochreinen Polycarbonaten nach dem Schmelze-Umesterungsverfahren aus DPC und Bisphenolen. Der bei der Reaktion gewonnene Chlorwasserstoff kann einem oder mehreren Reinigungsschritten unterzogen werden, sodass er für eine Vielzahl weiterer Verwendungsmöglichkeiten geeignet ist, insbesondere für elektrochemische oder thermische Oxidationen zu Chlor. Dieses so gewonnene Chlor kann zur Phosgenherstellung mit Kohlenmonoxid verwendet werden; das erhaltene Phosgen kann im erfindungsgemäßen Verfahren eingesetzt werden. Das unter den Reaktionsbedingungen flüssige Endprodukt wird in mehreren Destillationsschritten von Nebenprodukten gereinigt und weist einen Gehalt an mehr als 99,8% DPC auf. Der in der Reaktion verwendete Katalysator kann dabei so aufgearbeitet werden, dass er zu 50 Gew.% oder mehr in die Reaktion zurückgeführt werden kann.

Verfahren zur Herstellung von reinen Diarylcarbonaten aus Monohydroxyaromaten und Phosgen sind bekannt.

Die Herstellung von Diarylcarbonaten (wie z. B. Diphenylcarbonat) erfolgt üblicherweise durch ein kontinuierliches Verfahren, durch Herstellung von Phosgen und anschließende Reaktion von Monophenolen und Phosgen in einem inerten Lösungsmittel in Gegenwart von Alkali und einem Stickstoffkatalysator in der Grenzfläche.

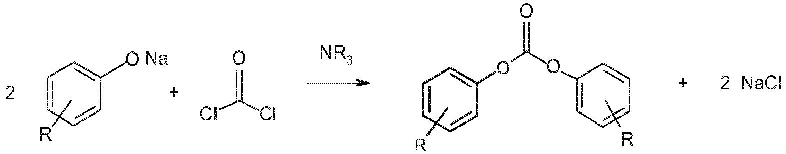

Die Herstellung von Diarylcarbonaten z.B. durch den Phasengrenzflächenprozess ist prinzipiell in der Literatur beschrieben, siehe z.B. in Chemistry and Physics of Polycarbonates, Polymer Reviews, H. Schnell, Vol. 9, John Wiley and Sons, Inc. (1964), S. 50/51.

Dabei werden nach dem Phasengrenzflächen-Verfahren die in Lösemitteln und Wasser gelösten Edukte miteinander umgesetzt. Der Nachteil dieser Verfahren besteht in der destillativen Abtrennung des Diarylcarbonats vom Lösemittel und dessen Wiederaufarbeitung, sowie in der Kochsalz-haltigen wässrigen Phase als Zwangsanfall-Produkt, für die es nur begrenzte Verwendungsmöglichkeiten gibt und die gegebenenfalls sehr aufwendige Aufarbeitungsschritte erfordert.

Daher wurden Verfahren zur sogennanten Direkt-Phosgenierung von Monohydroxyaromaten entwickelt, bei denen die Edukte Phosgen und Monohydroxyaromat in der Schmelze in Gegenwart von Katalysatoren, jedoch ohne Verwendung von Lösemitteln, zu Diarylcarbonaten und Chlorwasserstoff umgesetzt werden.

So wird beispielsweise in der US2,362,865 (A) ein Verfahren zur Herstellung von Diarylcarbonaten durch Direkt-Phosgenierung von Monophenolen bei Temperaturen von 170°C bis 250°C unter Verwendung von Al- oder Ti-Phenolaten beschrieben, wobei aber keine Rückführung des Katalysators, geschweige denn einen Einfluss der Katalysator-Rückführung auf die Bildung von o-Hydroxy-Benzoesäure-Derivate beschrieben ist.

Sowohl in der zum Zeitpunkt dieser Anmeldung unveröffentlichten EP 10158364.9 als auch in der unveröffentlichten EP 10158446.4 werden ebenfalls Verfahren zur Herstellung von Diarylcarbonaten durch Direkt-Phosgenierung von Monophenolen bei Temperaturen von 20°C bis 240°C unter Verwendung von Metall-Halogeniden oder Metall-Phenolaten beschrieben. Eine Rückführung des Katalysators in den Prozess ist nicht beschrieben worden. Auch in diesen Anmeldungen gibt es weder Hinweise auf die Bildung von o-Hydroxy-Benzoesäure-derivaten als Nebenprodukte, wie z.B. Salol, noch gibt es Angaben zu Maßnahmen, um die Bildung solcher Nebenprodukte zu verringern.

In der EP 1 234 845 A wird ebenfalls die Umsetzung von Monophenolen in der Schmelze bei Temperaturen von 120°C bis 190°C mit einem besonders reinen Phosgen beschrieben. Als Katalysatoren werden Stickstoff-haltige Verbindungen, wie z.B. Pyridin in Mengen von 0,1 bis 10mMol% bezogen auf eingesetztes Monophenol verwendet. Auch diese Veröffentlichung gibt keine Hinweise auf eine Rückführung von Katalysator in den Prozess. Es bleibt zudem offen, in welcher Menge o-Hydroxy-Benzoesäure-derivate als Nebenprodukte gebildet werden, und durch welchen Einfluss die Rückführung des Katalysators auf die Bildung von O-Hydroxy-Benzoesäure-Derivate hat.

Darüber hinaus gibt es eine Anzahl weiterer Schutzrechte, wie z.B. WO 2008/114750 A1, JP 2008-231073 A, JP 2009-114195 A, JP 09-278714 A, JP 09-100256 A, EP 1657272 A1, in denen die Umsetzung von Monophenolen bei höheren Temperaturen in der Schmelze mit Phosgen in Gegenwart homogen löslicher Stickstoff-haltiger Katalysatoren zu Diarylcarbonaten beschrieben wird.

In der WO 95/25083 A1, sowie in der EP 567 677 A1 werden jeweils im Reaktionsmedium homogen lösliche Phosphorverbindungen, wie z.B. Phosphite oder Phosphine als Katalysatoren in Direktphosgenierungsreaktionen von Monophenolen eingesetzt. Auch hier gibt es keine Hinweise auf eine mögliche Rückführung des Katalysators sowie dem Einfluss der Rückführung auf o-Hydroxy-Benzoesäure-derivate als Nebenprodukte der Phosgenierungsreaktion oder auf Einflüsse der eingesetzten Katalysatoren auf die Bildung solcher Nebenprodukte.

Die aus dem Stand der Technik bekannten Verfahren reichen nicht, um den hohen ökonomischen und ökologischen Anforderungen gerecht werden und darüber hinaus hohe Reinheiten der Produkte sicherstellen, die ihrerseits Einsatzstoffe für weitere chemische Prozesse sind, bei denen Nebenprodukte stören würden.

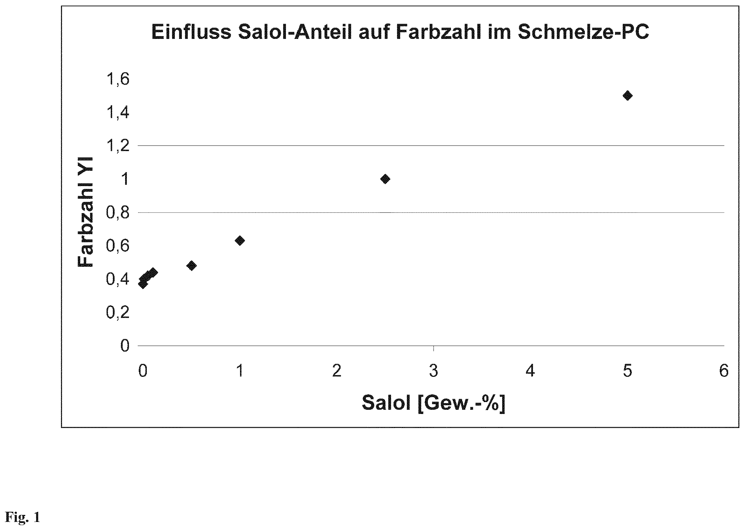

Für DPC gibt es wichtige Reinheitskriterien hinsichtlich der weiteren Verwendung zur Polykondensation mit Bisphenolen zu Polycarbonaten nach dem Umesterungsverfahren. Umesterungsfähige Nebenprodukte, wie z.B. Salicylsäurephenylester (Salol) können als artfremde Endgruppen in die Polymerkette eingebaut werden und unvorhersehbare Nebenwirkungen haben. Beispielsweise hat Salol einen direkten Einfluss auf die Qualität des daraus hergestellten Polycarbonats, wie aus Fig. 1 zu entnehmen ist. Solche Verbindungen werden im Folgenden mit dem allgemeinen Begriff "o-Hydroxy-Benzoesäure-Derivate" bezeichnet; dies bedeutet, dass es sich um einen ein- oder mehrkernigen aromatischen Kohlenstoffkern handelt, der neben der Hydroxyl-Gruppe in ortho-Stellung zu einer Carbonsäureester-Gruppe noch weitere Substituenten am aromatischen Kern enthalten kann. Ein möglichst geringer Gehalt solcher Verbindungen im Diarylcarbonat ist daher erstrebenswert.

Jedoch hat die Direktphosgenierung, das heisst die Phosgenierung von Phenol in Abwesenheit von Lösemittel, gerade den Nachteil, im Gegensatz zur Phosgenierung von Phenol in Anwesenheit von Lösemittel, der Bildung dieser o-Hydroxy-Benzoesäure-Derivate.

Daher ist in einem Direktphosgenierungsverfahren eine Rückführung des Katalysators unerwünscht, da dadurch zwangsweise o-Hydroxy-Benzoesäure-Derivate im Kreis gefahren werden und das Produkt (Diarylcarbonat) verunreinigt wird, so, dass ein Fachmann angesichts der vorliegenden Aufgabe keine Rückführung des Katalysators in Betracht ziehen würde.

Neben den Reinheitskriterien für die Produkte ist auch die Effizienz des Herstellverfahrens ein wichtiges Verfahrensmerkmal. So ist z.B. der Umsatz von Phosgen mit Phenol in der kondensierten homogenen Phase aufgrund der erhöhten Konzentration vom Phosgen im Phenol deutlich höher, als im zweiphasigen Gemisch von gasförmigem Phosgen und flüssigem Phenol. Auch die Temperaturerhöhung wirkt beschleunigend auf die Reaktionsgeschwindigkeit, sodass erhöhte Temperaturen im Bereich von 100°C bis 250°C, vorzugsweise 110°C bis 220°C von Vorteil sein können. Solche Temperaturen wirken allerdings der Löslichkeit von Phosgen in Phenol entgegen, sodass eine Reaktionsführung bei erhöhter Temperatur unter Druck besonders vorteilhaft ist.

Daher stellte sich die technische Aufgabe ein Verfahren zur Herstellung von Diarylcarbonaten nach dem Direktphosgierungsverfahren in der Schmelze eines Monophenols unter Verzicht auf zusätzliche Lösemittel bei erhöhter Temperatur und gegebenenfalls bei erhöhtem Druck zu entwickeln, das durch Reduzierung von Ausschleusen von Strömen (Purge) ökonomisch betrieben wird und bei gleichbleibend guter Qualität der Endprodukte, insbesondere durch Bereitstellung eines Diarylcarbonats mit einem Gehalt an o-Hydroxy-Benzoesäure-Derivaten (wie z.B. Salol) von weniger als 1,5 Gew% , vorzugsweise von weniger als 1,0 Gew% bereitgestellt.

Die technische Aufgabe konnte überraschenderweise dadurch gelöst werden, dass der Katalysator in die Reaktion der Umsetzung zu Diarylcarbonaten zurückgeführt wird.

Das erfindungsgemäße Verfahren besteht aus den drei Verfahrens-Abschnitten:
A) Reaktion,
B) Chlorwasserstoff-Aufarbeitung (optional)
C) Diarylcarbonat-Aufarbeitung

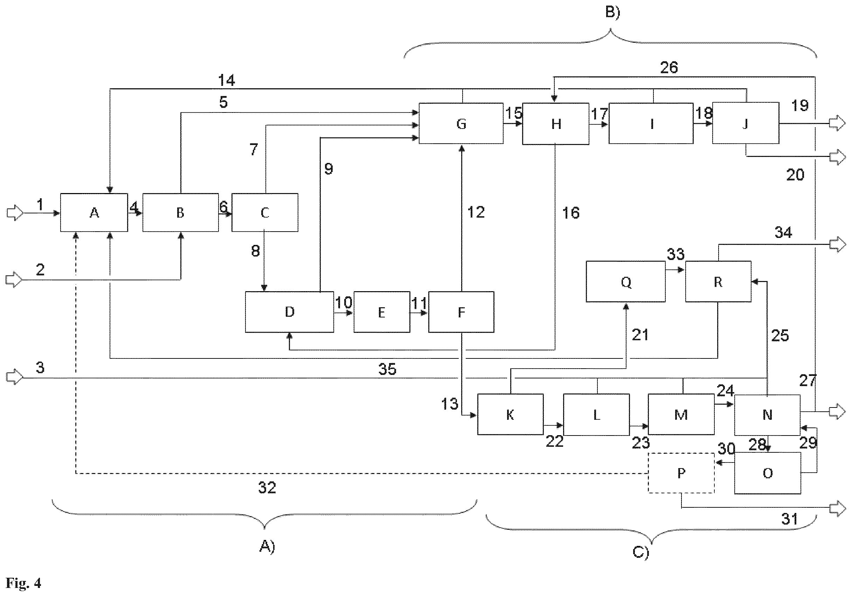

Eine graphische Gesamtübersicht gibt die Figur 4. (Gesamtverfahren)

In dem Verfahrensabschnitt A), Reaktion, werden die Edukte in einem vorgelagerten Verfahrensschritt in der Weise mit einander vermischt, dass eine weitgehend homogene Lösung von Phosgen im geschmolzenen Monophenol vorliegt; dies kann gegebenenfalls durch Anwendung erhöhter Drücke bei den vorgegebenen Schmelzetemperaturen erfolgen. Der Begriff "Monophenol" wird im Folgenden für alle aromatischen Mono-Hydroxy-Verbindungen benutzt, wobei es sich um ein- oder mehrkernige substituierte oder unsubstituierte aromatische Mono-Hydroxy-Verbindungen handeln kann. Geeignete Monophenole für das erfindungsgemäße Verfahren sind aromatische Verbindungen, die eine OH-Gruppe an einem cyclischen aromatischen Kohlenstoffkern gebunden enthalten. Es kann sich dabei um ein- oder mehrkernige aromatische Kohlenstoffverbindungen oder ein- oder mehrkernige heterozyklische Verbindungen mit beispielsweise ein oder mehreren Stickstoff- oder Phosphoratomen handeln. Diese aromatischen Verbindungen können weiterhin einen oder mehrere Substituenten an verschiedenen Positionen des aromatischen Kerns enthalten.

Im Rahmen der Erfindung geeignete aromatische(n) Hydroxyverbindung(en) (Monophenole) die in Schritt (h) verwendet werden sind bevorzugt solche der allgemeinen Formel (I) 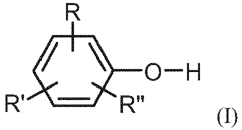 worin R, R' und R" unabhängig voneinander Wasserstoff, Halogen oder ein verzweigter oder unverzweigter Ci- bis C₉-Alkylrest oder Alkoxycarbonylrest sein können.

Solche aromatischen Monophenole sind beispielsweise: Phenol, o-, m- oder p-Kresol, auch als Gemisch der Kresole, Dimethylphenol, auch als Gemisch, wobei die Stellung der Methylgruppen am Phenolring beliebig sein kann, z.B. 2,4-, 2,6-, oder 3,4-Dimethylphenol, o-, m- oder p-Chlorphenol, 2,4-Dichlorphenol, p-Bromphenol und 2,4,6-Tribromphenol, o-, m- oder p-Ethylphenol, o-, m- oder p-n-Propylphenol, 4-iso-Propylphenol, 4-n-Butyl-phenol, 4-iso-Butylphenol, 4-tert-Butylphenol, 4-n-Pentylphenol, 4-n-Hexylphenol, 4-n-Octylphenol, 4-iso-Octylphenol, 4-n-Nonylphenol, 4-iso-Nonylphenol, o-, m- oder p-Methoxyphenol, 4-Cyclohexylphenol, 4-(1-Methyl-1-phenylethyl)-phenol, Biphenyl-4-ol, 1-Naphthol, 2-1-Naphthol, 4-(1-Naphthyl)phenol, 4-(2-Naph-thyl)-phenol, 4-Phenoxyphenol, 3-Pentadecylphenol, 4-Tritylphenol, Methylsalicylsäure, Ethylsali-cylsäure, n-Propylsalicylsäuret, iso-Propylsalicylsäure, n-Butylsalicylsäure, iso-Butylsalicylsäure, tert-Butylsalicylsäure, Phenylsalicylsäure, Benzylsalicylsäure und Salicylsäuremethylester.

Bevorzugte Monophenole sind Phenol, 4-tert-Butylphenol, Biphenyl-4-ol und 4-(1-Methyl-1-phenylethyl)-phenol.

Besonders bevorzugt ist Phenol.

Die eingesetzten Monophenole sollten eine Reinheit von mindestens 99,90 Gew% aufweisen. Bevorzugt enthalten die Edukte weniger als 300 vol. ppm Wasser da die Anwesenheit von Wasser die Korrosion der Apparatematerialien begünstigt.

Das hier eingesetzte Monophenol kann neben dem von außen in den Gesamtprozeß eingeführte Phenol, dem sogenannten Frischphenol aus Vorratstanks, auch rückgeführtes Monophenol aus Kondensatströmen der Verfahrensschritte B) und C) oder aus Waschflüssigkeitsströmen des Verfahrensschritts B) enthalten. Derartig rückgeführtes Monophenol kann Nebenprodukte aus dem Verfahren enthalten, wie z.B. Restmengen an Diarylcarbonat, Chlorwasserstoff oder Arylchlorkohlensäureester, die für die Reaktion unschädlich sind. In der eingesetzten Mischung der Edukte liegt das Monophenol vorzugsweise in mehr als der stöchiometrisch erforderlichen Menge bezogen auf Phosgen vor. Das molare Verhältnis von Monophenol zu Phosgen kann von 1,5:1 bis 4:1 variieren, bevorzugt ist ein Molverhältnis von 2:1 bis 3:1, besonders bevorzugt ist ein Molverhältnis von 2.5:1 bis 3:1.

Das eingesetzte Phosgen sollte zur Vermeidung von unerwünschten Nebenprodukten in den Endprodukten des Herstellverfahrens eine Reinheit von wenigstens 99,80 Gew%, vorzugsweise von 99,96 Gew% aufweisen; der Gehalt an Tetrachlorkohlenstoff sollte kleiner als 50 vol. ppm, vorzugsweise kleiner als 15 vol. ppm sein.

Geeignete Katalysatoren für das erfindungsgemäße Verfahren sind solche, die sich unter den Reaktionsbedingungen im Reaktionsgemisch ohne Zuhilfenahme zusätzlicher Lösemittel homogen lösen. Solche Katalysatoren sind z.B. Metallsalze, wie z.B. Aluminiumhalogenide, Zirkonhalogenide oder Titanhalogenide. Besonders bevorzugte Katalysatoren sind Titantetrachlorid, Aluminiumtrichlorid, oder Zirkontetrachlorid. Ganz besonders bevorzugt ist Titantetrachlorid und Aluminiumtrichlorid. Die Metallchloride wandeln sich unter den Reaktionsbedingungen relativ schnell ganz oder teilweise in die entsprechenden Metallphenolate, wie z.B. Titantetraphenolat um, die dann die homogen gelöste aktive Katalysatorkomponente darstellen. Die erfindungsgemäß verwendeten Katalysatoren können in Mengen von 0,001 mol% bis 10 mol% , vorzugsweise in Mengen von 0,01 mol% bis 5 mol% und besonders bevorzugt in Mengen von 0,05 mol% bis 2 mol% bezogen auf vorhandenes Monophenol eingesetzt werden.

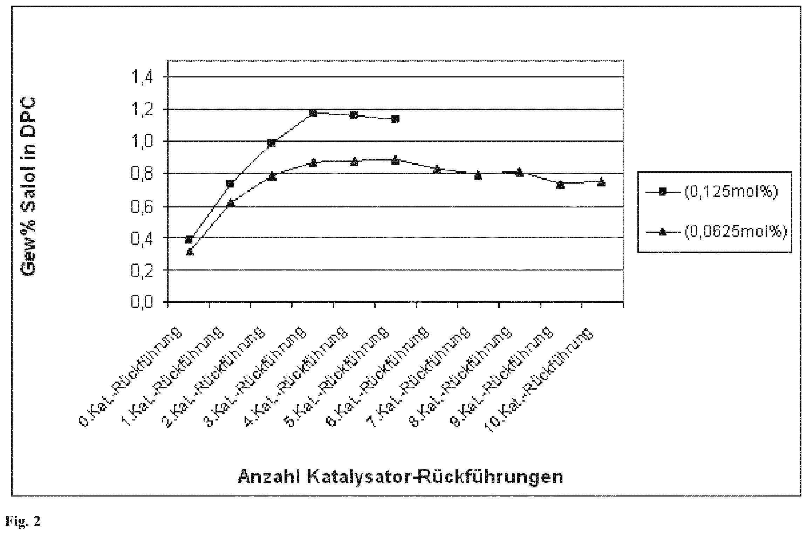

Überraschenderweise wurde gefunden, dass der Gehalt an o-Hydroxy-Benzoesäure-Derivaten im Diarylcarbonat auch bei wiederholtem Einsetzen des verwendeten Katalysators, der am Verfahrensende aus dem Prozess zurückgewonnen wird, nicht weiter steigen, sondern stagnieren (Plateau in Fig. 2). Eine solche Rückgewinnung des Katalysators kann beispielsweise durch Rückführung des Sumpfproduktes aus der letzten Diarylcarbonatdestillationsstufe erfolgen. Dieser wiederholte Einsatz von zurückgewonnem Katalysator im erfindungsgemäßen Diarylcarbonatherstellverfahren kann sowohl in diskontinuierlich als auch in kontinuierlich arbeitenden Verfahren erfolgen. Die Fig. 2 zeigt am Beispiel der Herstellung von Diphenylcarbonat in Gegenwart von Titantetrachlorid als Katalysator die Bildung von Salol als Nebenprodukt in Abhängigkeit von der Anzahl der wiederholten Einsätze von zurückgewonnenem Katalysator in die Reaktion. Dabei wird deutlich, dass unerwarteterweise bei wiederholtem Einsatz des Katalysators die Entstehung von Salol keine lineare Abhängigkeit zwischen Rückführung und Verunreinigung erfolgt, sondern ein Plateau erreicht wird, bei dem die Konzentration an o-Hydroxy-Benzoesäure-Derivaten nicht mehr ansteigt.

Die Katalysatoren werden vorzugsweise als Lösung in der Monophenol-Schmelze eingesetzt. Solche Lösungen können auch Katalysatormengen enthalten, die aus dem Verfahrensabschnitt C) der destillativen Diarylcarbonataufarbeitung als Diarylcarbonat-Sumpfprodukt anfallen und dann mit oder ohne gesonderte Katalysatoraufarbeitung in die Reaktion als Verfahrensabschnitt A) zurückgeführt werden. Eine Katalysatoraufarbeitung ist daher für die Rückführung des Katalysators in den Verfahrensabschnitt A) nicht zwangsweise erforderlich aber durchaus möglich. Die Zugabe der Katalysatoren erfolgt frühestens nach erfolgter vollständiger Durchmischung der Edukte vorzugsweise in dem Reaktor, um eine vorzeitige Reaktion der Edukte beim Vermischen und damit eine vorzeitige Chlorwasserstoffentwicklung in einem ungeeigneten Verfahrensabschnitt zu vermeiden.

Eine derartige Rückführung von Katalysatormengen aus dem Verfahrensabschnitt C) kann beliebig häufig vorgenommen werden; bei sogennanten Konti-Verfahren kann vorzugsweise eine Teilmenge des Katalysators kontinuierlich zurückgeführt werden, während eine Restmenge als Destillationssumpf aus dem Verfahrenskreislauf ausgeschleust wird. Bei Bedarf kann frischer Katalysator der zurückgeführten Katalysatormenge hinzugefügt werden. In das Verfahren werden wenigstens 50 Gew.% des Katalysators zurückgeführt, ganz besonders bevorzugt wenigstens 75 Gew.% und insbesondere ganz besonders bevorzugt wenigstens 85 Gew.%. In einer bevorzugten Ausführungsform werden jedoch maximal 99 Gew.% des Katalsyators zurückgeführt, bevorzugt 95 Gew.%

Die Konzentration des Nebenprodukts im Diarylcarbonat strebt überraschenderweise nach mehrmalig wiederholter oder längerer Katalysatorrückführung Grenzwerten entgegen, sodass bei lang anhaltender Rückführung des Katalysators kaum noch eine relative weitere Erhöhung der Nebenproduktkonzentration im Diarylcarbonat zu beobachten ist.

Falls eine Isolierung des Katalysators erwünscht ist, so ist sie abhängig von der Art des eingesetzten Katalysators und kann im Fall der Verwendung von Titan-tetrachlorid z.B. eine Phosgenierung des im Destillationssumpfes gebildeten Titantetramonophenolats bzw. Titan-Salolat zu Titantetrachlorid bedeuten. Auch Titanate mit Salol sind katalytisch aktiv. Bei Einsatz von Pyridin wäre das entstandene Pyridin-Hydrochlorid mit Basen in das freie Pyridin zu überführen.

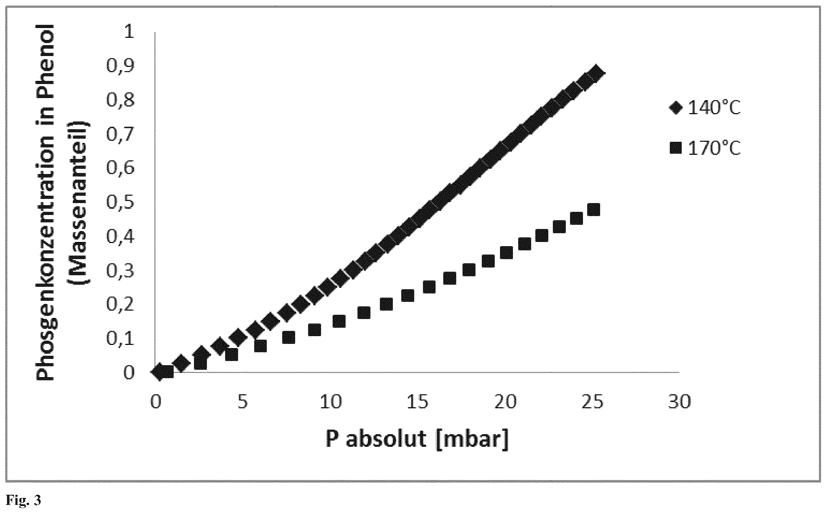

Die Edukte Monophenol und Phosgen werden in den oben angegebenen molaren Verhältnissen bzw. in den oben genannten bevorzugten molaren Verhältnissen mit einander gemischt, wobei das Monophenol stets als Schmelze vorliegt und das Phosgen je nach vorherrschendem Druck gasförmig oder flüssig ist. Bei Normaldruck und Temperaturen oberhalb von 60°C liegt weitgehend ein zweiphasiges Gas-Flüssigkeit-Gemisch vor, da die Löslichkeit von Phosgen auch in Monophenolen, ebenso wie in Diarylcarbonaten bei zunehmender Temperatur abnimmt, wie es in Fig. 3 für die Löslichkeit von Phosgen in Phenol veranschaulicht wurde.

Daher müssen Gemische aus geschmolzenen Monophenolen und Phosgen in der Reaktionsphase sehr intensiv gemischt und redispergiert werden, um über eine ausreichende Erneuerung der Phasengrenzflächen einen hinreichenden Umsatz der Edukte zu gewährleisten. Deshalb werden die Edukte bei erhöhter Temperatur unter Normaldruck, vorzugsweise unter erhöhtem Druck bis zu 50 bar (a) - wobei (a) den Absolutdruck bedeutet - besonders bevorzugt bei erhöhtem Druck bis 25 bar (a) und ganz besonders bevorzugt bei Drücken von 8 bis 25 bar (a) mit einander vermischt und zur Reaktion gebracht. Die Temperatur in der Mischzone sollte mindestens die Schmelztemperatur des Monophenols betragen, vorteilhaft aber eine Reaktionstemperatur im Bereich von 100°C bis 250°C erreichen.

Nach der weitgehend vollendeten Mischung der Edukte wird dem Gemisch vorzugsweise einer der oben genannten Katalysatoren vorzugsweise in der bevorzugten Menge als Lösung im Monophenol hinzugesetzt. Da bei den oben genannten Temperaturen und Drücken die katalysierte Umsetzung von Monophenol mit Phosgen zum Arylchlorkohlensäureester als Zwischenprodukt sehr rasch unter Abspaltung von gasförmigem Chlorwasserstoff verläuft, kann die Reaktion bevorzugt mehrstufig durchgeführt werden. Die Reaktion kann unter adiabatischen Bedingungen geführt werden, da sie nur eine leichte Wärmetönung aufweist. In einer ersten Stufe, dem sogenannten Hauptreaktor, entstehen insbesondere bei erhöhtem Druck und bevorzugt bei Temperaturen von 120°C bis 230°C, besonders bevorzugt bei Temperaturen von 130°C bis 210°C und für die Herstellung von Diphenylcarbonat ganz besonders bevorzugt bei einer Temperatur von 160°C bis 180°C und bei Reaktor-Verweilzeit von 5 bis 60 min , vorzugsweise von 5 bis 10 min überwiegend Arylchlorkohlensäureester neben bereits weiterreagiertem Diarylcarbonat. In einer zweiten Stufe reagiert in einem sogenannten Nachreaktor der Arylchlorkohlensäureester bei etwas höheren Temperaturen von vorzugsweise 170°C bis 250°C, besonders bevorzugt von 190°C bis 230°C und ganz besonders bevorzugt von 200°C bis 210°C bei Reaktorverweilzeiten von 5 bis 60 min, vorzugsweise von 5 bis 20 min mit noch vorhandenem Monophenol zum Diarylcarbonat. Dabei kann der Druck in der zweiten Stufe im sogenannten Nachreaktor auch auf 2 bis 20 bar abgesenkt werden. Eine derartige Absenkung des Druckes kann vorteilhaft in einer sogenannten Flash-Stufe vorgenommen werden, in der als Folge der Druckabsenkung das im Hauptreaktor entstandene Chlorwasserstoffgas besonders gut aus der Reaktionsschmelze abgetrennt werden kann. Auch hinter der zweiten Reaktionsstufe im Nachreaktor kann sich optional eine Flash-Stufe zur Abtrennung der Restmengen an Chlorwasserstoff befinden. Sie kann optional auch in die erste Destillationskolonne des nachfolgen Verfahrensabschnitts C), der destillativen Diarylcarbonataufarbeitung integriert sein und dort die Trennung von Gas- und Flüssigphase beschleunigen.

Als Reaktoren für die Umsetzung der Edukte unter den angegebenen Reaktionsbedingungen sind vorzugsweise Konti-Reaktoren gut geeignet, möglich ist aber auch die Verwendung von Rührkesseln als batch-Reaktoren. Besonders gut geeignete Konti-Reaktoren sind z.B. Rührkesselkaskaden, Blasensäulenkolonnen, Bodenkolonnen, Füllkörperkolonnen oder Kolonnen mit feststehenden Einbauten zur Durchmischung des Reaktionsmediums oder Reaktionsdestillationskolonnen.

Solche Kolonnen können auch mit einander kombiniert sein, wie z.B. eine Blasensäulenkolonne mit einer aufgesetzten Rektifikationskolonne, wobei abweichend von der oben beschriebenen Mischung der Edukte, die Edukte getrennt an unterschiedlichen Stellen der Kolonnenkombination eingeführt werden können. So kann z.B. in der oben genannten Kolonnenkombination das Phosgen in die untere Blasensäulenkolonne und das Monophenol zusammen mit dem Katalysator in die obere Rektifikationskolonne mit ca. 10 theoretischen Böden eingeführt werden. Das entstandene Diarylcarbonat wird aus der Blasensäulenkolonne entnommen.

Eine entsprechend getrennte Dosierung der Edukte kann auch in einer Reaktionsdestillationskolonne derart erfolgen, dass das Phosgen in der Mitte der Kolonne eingeführt wird und dass das Monophenol zusammen mit Katalysator am Kopf der Kolonne eingebracht wird. Das Reaktionsgemisch wird dem Kolonnensumpf entnommen. Solche Kolonnen können mindestens 5, vorzugsweise ca. 20 Böden aufweisen.

In einer weiteren optionalen Ausführungsform der Reaktoren können die Edukte in einem Hauptreaktor bei Drücken von 1 bis 25 bar (a) bei ausreichend hoher, gegebenenfalls längerer Verweilzeit aber geringeren Temperaturen im unteren Teil des Reaktors von vorzugsweise 120°C bis 190°C, besonders bevorzugt von 160°C bis 180°C vollständig umgesetzt werden. Im oberen Teil des Reaktors ist eine zusätzliche Beheizung erforderlich, um dort etwas höhere Temperaturen bis 250°C, vorzugsweise bis 230°C zu realisieren. Die weitgehende Entgasung des Reaktionsgemischs und Abtrennung der Leichtsieder kann anschließend durch eine FlashVerdampfung oder eine andere Entgasungstechnik erfolgen.

Besonders bevorzugt sind Blasensäulenkolonnen, die mit dem Eduktgemisch wie oben beschrieben von unten nach oben durchströmt werden. Dabei werden am Kolonnenkopf der Blasensäulenkolonne der gasförmige Chlorwasserstoff und am oberen Ende des Kolonnenschafts das Reaktionsgemisch entnommen. Dieses wird der nächsten Blasensäulenkolonne, die als Nachreaktor fungiert, über den Kolonnenboden zugeführt. Aus der letzten Blasensäulenkolonne wird das vollständig ausreagierte Reaktionsgemisch am Ende eines Verweilreaktors entnommen und dem nachfolgenden Verfahrensabschnitt C), der destillativen Diarylcarbonataufarbeitung zugeführt. Das jeweils am Kopf der Blasensäulenkolonnen entnommene Chlorwasserstoffgas wird in dem nachfolgenden Verfahrensabschnitt B), der Chlorwasserstoffaufarbeitung vereinigt. Eine zusätzliche Abtrennung von Chlorwasserstoff ist auch zwischen den einzelnen Stufen durch Entspannen in einem Flash und eine anschließende Druckerhöhung möglich.

Die Apparatematerialien müssen den hohen Anforderungen an Beständigkeit gegenüber Chlorwasserstoff und Phosgen bei hohen Temperaturen entsprechen und werden vorzugsweise ausgewählt aus der Gruppe der Werkstoffe Schwarzer Stahl, Edelstahl, Stahllegierungen, Nickel-Basislegierungen (z.B. Hastelloy C), Keramik, Graphit, Email-beschichtete Materialien, PTFEverkleidete Materialien.

In dem Verfahrensabschnitt B), (optional) der Chlorwasserstoffaufarbeitung, wird die in der Reaktion A) entstandene Gasphase gesammelt, von dem Hauptprodukt Chlorwasserstoffgas abgetrennt, und gegebenenfalls für eine weitere Umsetzung zu Diarylcarbonat unterzogen und in die Reaktion zugeführt. Das Hauptprodukt Chlorwasserstoff kann zur Erhöhung der Reinheit destilliert werden.

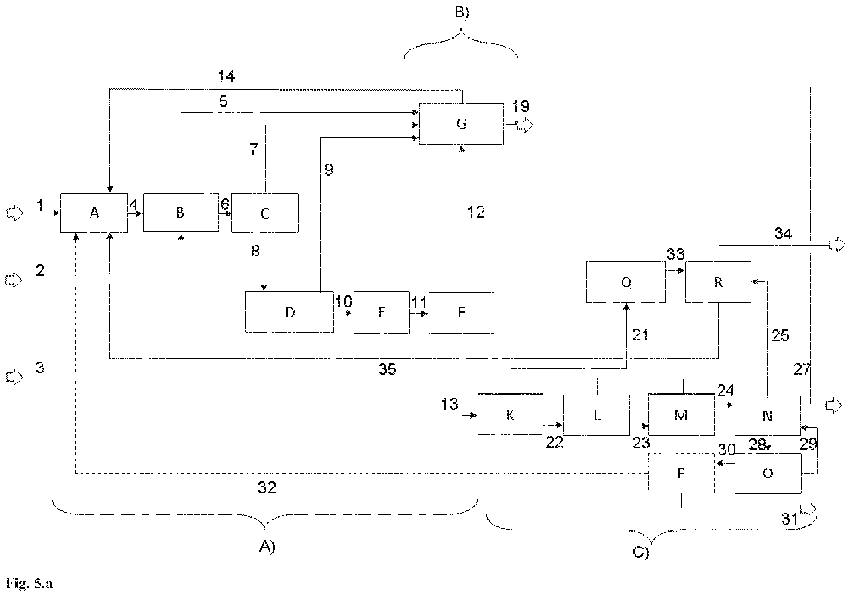

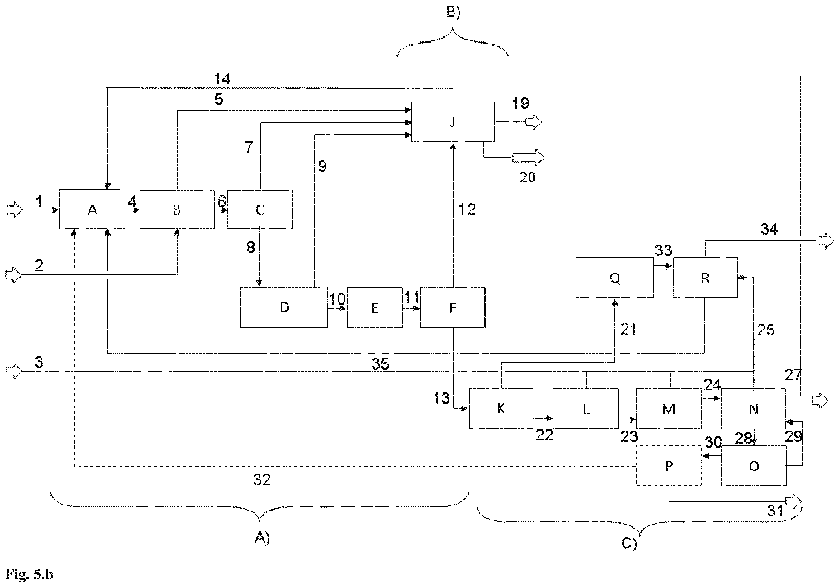

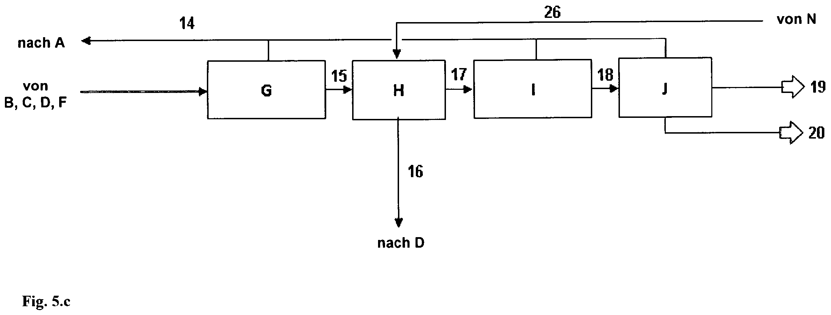

Eine graphische Übersicht dieses Verfahrensabschnitts gibt Fig. 5. (Chlorwasserstoffaufarbeitung)

In diesem Verfahrensabschnitt B werden mehrere Stoffströme aus dem Verfahrensabschnitt A) (Reaktion; s. Ströme 5,7 und 12) vereinigt und gemeinsam gereinigt. Hauptprodukt unter den leichtsiedenden Komponenten ist mit 90 Gew% oder mehr das Chlorwasserstoffgas; Nebenprodukte sind das im Überschuss eingesetzte Monophenol mit mehr als 5 Gew %, sowie Spuren an Arylchlorkohlensäureester, Diarylcarbonat und Phosgen und als Nebenprodukt aus dem Phosgen Spuren an Kohlenmonoxid und Tetrachlorkohlenstoff. Die Nebenprodukte können über verschiedene Schritte vom Hauptprodukt Chlorwasserstoff weitgehend abgetrennt werden, sodass ein Chlorwasserstoffgas mit einer Reinheit von mehr als 99,0 Vol.%, vorzugsweise von mehr als 99,9 Vol.% und einem Restgehalt an Phosgen und/oder Chlorkohlensäureestern von weniger als 100 vol. ppm, vorzugsweise von weniger als 10 vol. ppm und besonders bevorzugt von weniger als 10 ppb erhalten wird. Ebenso sollte der Gehalt an organischen Verbindungen im Chlorwasserstoff kleiner als 1000 vol. ppm, vorzugsweise kleiner als 50 vol. ppm sein, insbesondere der Gehalt an chlorhaltigen Kohlenwasserstoffen sollte kleiner 50 vol. ppm sein.

Diese Aufgabe wird durch einen oder mehrere Schritte gelöst, die nachfolgend beschrieben sind. Vorzugsweise wird diese Aufgabe durch einen mehrstufigen Prozess gelöst.

In einer sogenannten ersten Kondensationsstufe werden die Nebenprodukte mit einem höheren Siedepunkt als dem von Chlorwasserstoff bei geeigneter Temperatur auskondensiert. Dabei werden insbesondere höher siedende Komponenten, die in größerer Konzentration vorliegen, wie Monophenole und Diarylcarbonate weitgehend aus dem Chlorwasserstoffgas entfernt, die in die Reaktion zurückgeführt werden können. Diese Abtrennung gelingt besonders gut, wenn neben der tieferen Temperatur optional auch erhöhte Drücke angewendet werden. Bevorzugte Temperaturen in der ersten Kondensationsstufe sind mindestens 80°C, und für die Herstellung von Diphenylcarbonat besonders bevorzugt 90°C. Der Druck wird vorzugsweise in einem Bereich von 8 bis 25 bar (a) eingestellt, ein besonders bevorzugter Druck für die Herstellung von Diphenylcarbonat ist 12 bar (a). Die Kondensation der Nebenprodukte aus dem Chlorwasserstoffgasstrom kann optional auch mehrstufig bei verschiedenen Temperaturen und / oder Drücken erfolgen.

Falls eine ausreichend tiefe Temperatur oder ein ausreichend hoher Druck technisch nicht oder nur schwer zu realisieren ist, kann diese erste Kondensationsstufe auch übergangen werden, um die Nebenprodukte in einer nachfolgenden sogenannten HCl-Waschstufe in einer geeigneten Apparatur mit geschmolzenem Diarylcarbonat aus dem Chlorwasserstoffstrom heraus zu waschen. Stellt diese HCl-Waschstufe die erste Reinigungsstufe für den Chlorwasserstoff unter Umgehung der ersten Kondensationsstufe dar, so kann diese HCl-Waschstufe auch in sich mehrstufig ausgebildet sein und auf verschiedenen, fallenden Temperaturniveaus betrieben werden, um die Effizienz der Wäsche zu erhöhen. Dabei lösen sich insbesondere Monophenole sehr gut im Diarylcarbonat. Auch Spuren an Chlorkohlensäureester und Phosgen können in diesem Verfahrensschritt noch zum Diarylcarbonat umgesetzt werden, wenn das zur Wäsche eingesetzte Diarylcarbonat z. B. an einer geeigneten Stelle im nachfolgenden Verfahrensabschnitt C), der Diarylcarbonataufarbeitung entnommen wird. Prinzipiell ist jeder Diarylcarbonatstrom dieses Verfahrensabschnitts bis hin zum destillierten Diarylcarbonat für die HCl-Waschstufe geeignet, vorteilhaft für die Umsetzung der genannten organischen Chlorverbindungen kann es sein, einen Katalysator- und Phenol-haltigen Diarylcarbonatstrom für die HCl-Waschstufe dem Verfahrensabschnitt C) zu entnehmen, um in kurzer Zeit die im Chlorwasserstoffgas noch vorhandenen organischen Chlorverbindungen zur Reaktion bringen zu können.

Ein solches geeignetes Diarylcarbonat ist z.B. das Sumpfprodukt Strom 13 (s. Fig. 4, Gesamtverfahren) am Ende des Verfahrensabschnitts A) (Reaktion), das zur weiteren Aufarbeitung der ersten Stufe des Verfahrensabschnitts C) (Diarylcarbonataufarbeitung) zugeleitet wird. In diesem Diarylcarbonat sind ausreichende Mengen an Katalysator und an Phenol vorhanden. Alternativ-dazu kann ein destilliertes Diarylcarbonat in beliebiger Weise für die HCl-Waschstufe verwendet werden, da die physikalische Löslichkeit der auszuwaschenden Nebenprodukte im DPC hinreichend hoch ist. Bevorzugt jedoch wird ein reines destilliertes Diarylcarbonat für die HCl-Waschstufe verwendet, das dem Stoffstrom 27 (s.Fig 3, Gesamtverfahren) entnommen wird. Zur Umsetzung der organischen Chlorverbindungen in der HCl-Waschstufe kann anstelle des Diarylcarbonats als Waschmedium ebenso auch das Monophenol verwendet werden, da auch im Monophenol die physikalische Löslichkeit der auszuwaschenden Nebenprodukte hinreichend hoch ist. Dieses Monophenol kann z.B. ein Teilstrom des Monophenol-Eduktstromes sein. Falls eine Reaktion von Chlorestern oder Phosgen zu Diarylcarbonat erwünscht ist, so kann das zur Wäsche verwendete Monophenol in beliebiger Weise Katalysator enthalten. Die HCl-Wäsche mit Diarylcarbonat oder mit Monophenol wird vorzugsweise bei Temperaturen oberhalb des Schmelzpunktes des Diarylcarbonats durchgeführt; bei der Herstellung von Diphenylcarbonat wird eine Schmelzetemperatur von 95°C besonders bevorzugt. Die HCl-Wäsche kann bei Normaldruck oder bei erhöhtem Druck von 8 bis 25 bar (a) durchgeführt werden; bei der Herstellung von Diphenylcarbonat sind 12 bar (a) besonders bevorzugt.

Bei einer derartigen Wäsche kann ein Chlorwasserstoffgas mit einer Reinheit von 99,9 Gew% erhalten werden. Der Anteil an Phosgen liegt unter 50 vol. ppm, der von Chlorameisensäureester liegt unterhalb der Nachweisgrenze und der Phenolgehalt wird bis auf unter 1 vol. ppm verringert.

Diese HCl-Waschstufe ist nicht zwingend erforderlich und kann bei beliebiger Kombination anderer Verfahrensschritte miteinander auch umgangen werden.

Zur Erzielung hoher Reinheiten des Chlorwasserstoffgases ist eine Chlorwasserstoffdestillation besonders gut geeignet. Um eine solche Destillation energieeffizient durchführen zu können, ist die vorangehende Abkühlung des zu reinigenden Chlorwasserstoffs auf tiefere Temperaturen in einer vorgeschalteten zweiten Kondensationsstufe sinnvoll, aber nicht zwingend erforderlich. Entfällt diese Stufe, dann ist in der nachfolgenden Chlorwasserstoffdestillation eine entsprechend höhere Energie bei tiefen Temperaturen erforderlich. In dieser zweiten Kondensationsstufe, die optional auch auf mehreren unterschiedlichen Temperatur- und /oder Druckniveaus arbeiten kann, werden die im Chlorwasserstoffgas noch enthaltenen Spuren an höher siedenden Nebenprodukten abgeschieden, insbesondere bei Anwendung höherer Drücke im Bereich von 8 bis 25 bar (a), bei Diphenylcarbonat bevorzugt 12 bar (a). Die Temperaturen können in Abhängigkeit von den technischen Gegebenheiten in einem sehr weiten Bereich von plus 25°C bis minus 50°C variieren. Diese zweite Kondensationsstufe ist insbesondere dann sehr empfehlenswert, wenn die Wäsche in der HCl-Waschstufe mit Monophenol durchgeführt wurde, da auf diese Weise die im HCl-Gasstrom vorhandene Konzentration an Monophenol deutlich abgesenkt werden kann und damit die HCl-Destillation entlastet wird. Entfällt diese zweite Kondensationsstufe, so sind die Anforderungen an den Energiebedarf in der HCl-Destillation entsprechend sehr viel höher. Die Kondensate können ebenfalls, wie in der ersten Kondensationsstufe der Reaktion zugeführt werden.

Als vierte und letzte Stufe der Chlorwasserstoffaufarbeitung im Verfahrensabschnitt B) ist die Chlorwasserstoffdestillation in einer besonders bevorzugten Ausführungsform besonders gut geeignet zur Herstellung eines hochreinen Chlorwasserstoffs. Sie sollte vorzugsweise bei erhöhtem Druck durchgeführt werden, da sonst der energetische Aufwand für das Einstellen alternativ erforderlicher hinreichend tiefer Temperaturen unverhältnismäßig hoch wäre. Sollten vorangegangene Reinigungsstufen bei Normaldruck durchgeführt worden sein, so ist spätestens bei dieser Reinigungsstufe eine Verdichtung des Chlorwasserstoffstromes auf höhere Drücke von 8 bis 25 bar (a) sehr empfehlenswert; für die Herstellung von Diphenylcarbonat sind 12 bar (a) besonders bevorzugt. Unter diesen Bedingungen ist ein Chlorwasserstoffgas mit einer Reinheit von 99,95 Gew % erhältlich.

Alle der vier oben genannten Stufen der Chlorwasserstoffreinigung im Verfahrensabschnitt B) sind in der beschriebenen Reihenfolge erfindungsgemäß besonders gut zur Herstellung eines hochreinen Chlorwasserstoffgases geeignet. Die Einhaltung bestimmter Reihenfolgen oder die Durchführung aller Verfahrensstufen ist nicht zwingend erforderlich, sondern hängt ab von dem Grad der Verunreinigung des aus der Reaktion abgeschiedenen Chlorwasserstoffs und von dem gewünschten Reinheitsgrad des Chlorwasserstoffgases als Endprodukt. So ist es gegebenenfalls durchaus möglich, mit einzelnen Reinigungsstufen oder einer einzigen Reinigungsstufe das gewünschte Ergebnis zu erzielen, wie im Folgenden am Beispiel der HCl-Destillation dargestellt.

Werden die Eingangsstoffströme aus dem Verfahrensabschnitt A) (Reaktion; s. Ströme 5,7 und 12) ohne vorherige Reinigung direkt der Chlorwasserstoffdestillation zugeführt, so ist unter den gleichen Temperatur- und Druckbedingungen ebenfalls ein Chlorwasserstoffgas mit eine Reinheit von 99,95 Gew% erhältlich.

Eine Kombination der Reinigungsstufen ist durchaus in einer bestimmten, jedoch von oben genannter Aufzählung unabhängigen Reihenfolge zur Erzielung bestimmter Reinheitsgrade ausführbar.

Als Apparate zur Durchführung der ersten und zweiten Kondensationsstufe sind klassische Kühlfallen mit einer für die Prozessbedingungen ausreichend hohen Wärmetauscheroberfläche und einer Vorrichtung zur Ausspeisung der Kondensate in die Reaktion geeignet. Solche Kühlfallen können auch mehrstufig ausgeführt und gegebenenfalls verschieden temperiert sein. Geeignete Apparate für die HCl-Waschstufe sind insbesondere kontinuierlich betriebene Apparate, wie z.B. Blasensäulenkolonnen, Glockenbodenkolonnen, Füllkörpekolonnen, Packungskolonnen, Kolonnen mit feststehenden Einbauten, in denen die Waschflüssigkeit von oben dem aufsteigenden Chlorwasserstoffgas entgegen geführt werden. Auch kontinuierlich betriebene Rührapparate, wie z.B. Mixer-Settler oder auch diskontinuierlich betriebene Rührapparate sind prinzipiell geeignet.

Die Chlorwasserstoffdestillation kann in üblichen Destillations- oder Rektifikationskolonnen mit geeigneten Kolonneneinbauten vorgenommen werden.

Die Materialien für die oben genannten Apparate müssen den hohen Anforderungen an Beständigkeit gegenüber Chlorwasserstoff bei hohen Temperaturen entsprechen und werden vorzugsweise ausgewählt aus der Gruppe Schwarzer Stahl, Edelstahl, Stahllegierungen, Nickel-Basislegierungen (z.B. Hastelloy C), Keramik, Graphit, Email-beschichtete Materialien, PTFEverkleidete Materialien.

Im Verfahrensabschnitt C) der Diarylcarbonataufarbeitung werden die in der Reaktion A) entstandenen höher siedenden Komponenten gesammelt, getrennt und gegebenenfalls in die Reaktion zurückgeführt; das Hauptprodukt wird dabei so weit gereinigt, dass ein Diarylcarbonat mit einer Reinheit von mehr als 99,0 Gew%, vorzugsweise von mehr als 99,8 Gew% erhalten wird.

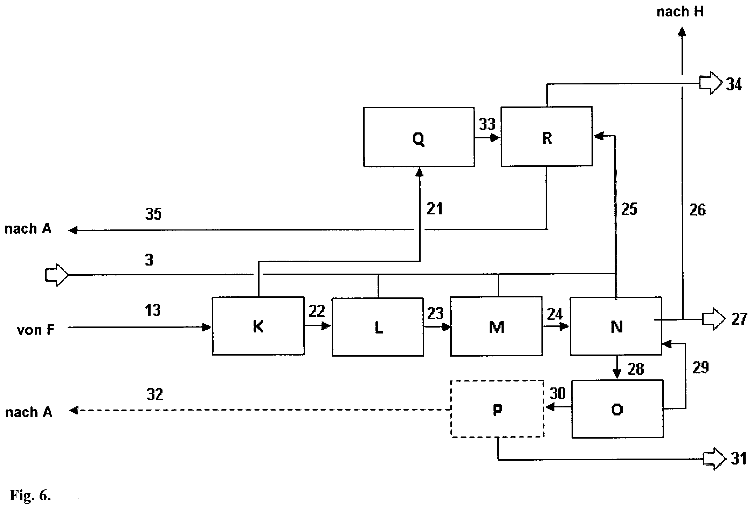

Eine graphische Übersicht dieses Verfahrensabschnitts gibt Fig. 6. (Diarylcarbonataufarbeitung)

Dazu wird das flüssige Reaktionsprodukt, als Sumpfprodukt aus dem Verweilreaktor bzw. als Sumpfprodukt aus der optional darauf folgenden Flashstufe des Verfahrensabschnitts A) in die erste Destillationsstufe des Verfahrensabschnitts C) überführt. Das flüssige Reaktionsprodukt enthält als Hauptprodukt das Diarylcarbonat und als wesentliche Nebenkomponente Monophenol, sowie wenige Gew% an Katalysator und an Chlorwasserstoff und wird in der ersten Destillationsstufe bei Temperaturen von max. 250°C, bevorzugt von 180°C bis 210°C und Drücken von 0,5 bis 3,0 bar (a), vorzugsweise von 0,8 bis 1,0 bar (a) von Chlorwasserstoff befreit. Dieser Chlorwasserstoff wird der ersten Kondensationsstufe im Verfahrensabschnitt B), der Chlorwasserstoffaufarbeitung zugeführt.

Anstelle einer Destillation als erster Verfahrensstufe kann auch eine Monophenol-Flashverdampfung verwendet werden, die gegebenenfalls auch mehrstufig ausgeführt sein kann. Aufgabe dieser Flashverdampfung, die in einer bevorzugten Ausführungsform die zweite Verfahrensstufe darstellt, ist die weitgehende Abtrennung von Phenol aus dem Hauptprodukt Diarylcarbonat, um die nachfolgende Monophenol-Destillationsstufe zu entlasten. Unter den Flashbedingungen von 150°C bis 250°C, vorzugsweise von 170°C bis 200°C bei einem Druck von 0,1 bis 1,0 bar (a), vorzugsweise 0,2 bis 0,5 bar (a) wird neben der Verdampfung von Monophenol auch eine weitgehende Verdampfung des Chlorwasserstoffs bewirkt. Die abgeschiedenen Monophenol-Brüden können direkt ohne weitere Reinigung in die Reaktion zurückgeführt werden. Chlorwasserstoffgas wird der ersten Kondensations-Stufe im Verfahrensabschnitt B), der Chlorwasserstoffaufarbeitung zugeführt.

Anstelle der Flashverdampfung, als zweiter Verfahrensstufe nach der ersten Destillationsstufe zur Entfernung von Chlorwasserstoff, kann auch eine zweite klassische Destillation des Sumpfproduktes aus der ersten Destillationsstufe zur weitgehenden Entfernung von Monophenol unter ähnlichen Bedingungen, wie oben für die Flashverdampfung beschrieben, erfolgen. Es ist erstrebenswert, spätestens bis zur zweiten Verfahrensstufe im Verfahrensabschnitt C) das flüssige Diarylcarbonatsumpfprodukt unabhängig von der Art der Verdampfungstechnik vollständig von Spuren an Chlorwasserstoff zu befreien.

Das Sumpfprodukt der zweiten Verfahrensstufe, das frei von Chlorwasserstoff ist und nur noch wenig Monophenol sowie Katalysatorbestandteile enthält, wird in der nachfolgenden dritten Verfahrensstufe, der Monophenoldestillation, von leichtsiedenden Nebenprodukten, im wesentlichen Monophenol, fast vollständig befreit. Die Destillation erfolgt in üblichen Destillations- oder Rektifikationskolonnen bei Sumpftemperaturen von bis zu 250°C, vorzugsweise von 180°C bis 210°C und einem Druck von weniger als 50 mbar (a), vorzugsweise bei 10 bis 30 mbar (a). Diese Monophenolbrüdenströme können ohne weitere Reinigung direkt der Reaktion zugeführt werden.

Dieses Sumpfprodukt aus der dritten Verfahrensstufe wird in der anschließenden vierten Verfahrensstufe, der Diarylcarbonatdestillation, bei Sumpftemperaturen von max. 210°C und einem Druck von weniger als 15 mbar (a) von Schwersiedern, wie z.B. den Katalysatorbestandteilen, durch Destillation befreit. Bei einer solchen Destillation kann das hochreine Diarylcarbonat mit einem Gehalt von mehr als 99,5 Gew%, vorzugsweise von mehr als 99,8 Gew% z.B. im Seitenstrom oder über Kopf der Destillationskolonne entnommen werden. Das so gewonnene Diarylcarbonat als Hauptprodukt des Herstellverfahrens enthält noch Spuren an ortho-Hydroxy-Benzoesäurederivaten in Mengen von weniger als 0,5 Gew%, vorzugsweise von weniger als 0,2 Gew%, da wegen der geringen Siedepunktsdifferenzen zum Diarylcarbonat eine vollständige destillative Abtrennung dieser Nebenprodukte vom Hauptprodukt technisch sehr schwierig ist. Die Konzentration dieser Nebenprodukte im destillierten Diarylcarbonat verringert sich durch Komplexbildung z.B. mit Titankatalysatoren, wodurch diese Nebenprodukte dann als Hochsieder im Destillationssumpf verbleiben. Am Kopf dieser Kolonne werden noch Spuren an Leichtsiedern abgeführt und der Reaktion zugeleitet. Bei den bevorzugten Sumpftemperaturen von maximal 210°C zur Vermeidung von Zersetzungsreaktionen in größerem Ausmaß im Kolonnensumpf verbleiben gegebenenfalls nicht unerhebliche Mengen an Diarylcarbonat im Sumpf der Kolonne zurück, die die Ausbeute an Diarylcarbonat mindern.

Daher kann der Sumpf der Diarylcarbonatdestillationskolonne optional einer fünften Verfahrensstufe, dem Diarylcarbonatverdampfer, zugeführt werden, in dem nach dem Prinzip von Dünnschichtverdampfern bei kurzen Verweilzeiten, einem Vakuum von vorzugsweise weniger als 15 mbar (a) und bei Temperaturen von mehr als 210°C eine ausreichende Menge Diarylcarbonat aus dem Sumpfprodukt der vierten Verfahrensstufe zurückgewonnen wird. Dieses so zurückgewonnene Diarylcarbonat, das noch Spuren an höher siedenden Nebenprodukten enthalten kann, wird vorzugsweise für eine weitere Reinigung in die Diarylcarbonatdestillationskolonne der vierten Verfahrensstufe zurückgeführt. Das jetzt noch verbleibende Sumpfprodukt in der fünften Verfahrensstufe enthält zum großen Teil Katalysatorbestandteile, die je nach eingesetztem Katalysator noch katalytisch aktiv sind und direkt oder nach einer weiteren Aufarbeitung erneut verwendet und ganz oder teilweise in die Reaktion zurückgeführt werden können. Bei Verwendung von Titantetrachlorid als Katalysator bildet sich das Titantetramonophenolat bzw. der gemischte Ester mit den ortho-Hydroxy-Benzoesäurederivaten, die als Nebenprodukte entstehen und ebenfalls an das Titan gebunden werden und als Hochsieder im Sumpf der Destillationen verbleiben.

Alle fünf der oben genannten Stufen der Diarylcarbonataufarbeitung im Verfahrensabschnitt C) sind in der beschriebenen Reihenfolge erfindungsgemäß besonders gut zur Herstellung eines hochreinen Diarylcarbonats geeignet. Die Einhaltung bestimmter Reihenfolgen oder die Durchführung aller Verfahrensstufen ist aber nicht zwingend erforderlich, sondern hängt ab von dem Anteil an Überschüssen des Monophenols, der Art des Katalysators und der Menge an Nebenprodukten in dem aus der Reaktion erhaltenen Reaktionsgemisch, sowie von dem gewünschten Reinheitsgrad des Diarylcarbonats als Endprodukt. So ist es gegebenenfalls durchaus möglich, mit einzelnen Reinigungsstufen oder einer einzigen Reinigungsstufe das gewünschte Ergebnis zu erzielen. Auch hier ist eine Kombination der Reinigungsstufen durchaus in einer bestimmten, jedoch von oben genannter Aufzählung unabhängigen Reihenfolge zur Erzielung bestimmter Reinheitsgrade ausführbar.

Geeignete Destillationsapparate im Verfahrensabschnitt C) der Diarylcarbonataufarbeitung sind z.B. Glockenbodenkolonnen, Füllkörperkolonnen, Packungskolonnen, Trennwandkolonnen, Kolonnen mit geeigneten Einbauten zur Erhöhung der Flüssigkeitsoberfläche, Dünnschichtverdampfer oder Flashverdampfer.

Der Unterdruck für die Destillationsstufen wird üblicherweise durch Vakuum-Flüssigkeitsringpumpen erzeugt. Er kann ebenso auch durch Dampfstrahler erzeugt werden, die mit dem in diesem Verfahren erzeugten Diarylcarbonat oder mit dem in diesem Verfahren verwendeten Phenol betrieben werden.
- **Fig. 1**: Einfluss der Salolkonzentration auf die Farbzahl des Polycarbonats
- **Fig. 2**: zeigt den Zusammenhang zwischen Anzahl Katalysatorrückführung und Salolkonzentration für den Einsatz von zwei verschiedenen Mengen an Titantetrachlorid bei der Synthese von Diphenylcarbonat aus Phenol und Phosgen.
- **Fig. 3**: zeigt die Löslichkeit von Phosgen in Phenol.
- **Fig. 4**: Übersicht über das Gesamtverfahren.
- **Fig. 5.a**: zeigt eine graphische Übersicht der Chlorwasserstoffaufarbeitung, 2-stufige Kondensation
- **Fig. 5.b**: zeigt eine graphische Übersicht der Chlorwasserstoffaufarbeitung (nur Destillation)
- **Fig. 5.c**: Chlorwasserstoffaufarbeitung (alle Stufen)
- **Fig. 6**: zeigt eine graphische Übersicht der Diarylcarbonataufarbeitung (Schritt C)

In den Figuren 4, 5.a und 5.b haben die Buchstaben mit Klammer folgende Bedeutung:
A) Ist der Verfahrensabschnitt "Reaktion"
B) Ist der Verfahrensabschnitt "Chlorwasserstoffaufarbeitung"
C) Ist der Verfahrensabschnitt "Diarylcarbonataufarbeitung".

Die Buchstaben in den rechteckigen Kästen haben die folgende Bedeutung:
- A:: Edukt-Mischaggregat
- B:: Hauptreaktor
- C:: Entgasungsstufe
- D:: Nachreaktor
- E:: Verweilreaktor
- F:: Entgasungs-oder Flashstufe
- G:: Erste Kondensationsstufe
- H:: Chlorwasserstoffwäsche
- I:: Zweite Kondensationsstufe
- J:: Chlorwasserstoffdestillation
- K:: Erste Destillation (Leichtsiederdestillation)
- L:: Flashverdampfung
- M:: Monophenoldestillation
- N:: Diarylcarbonatdestillation
- O:: Diarylcarbonatverdampfer (Dünnfilmverdampfer)
- P:: (optional) Katalysatorlösungsvorlage
- Q:: Kondensator
- R:: Leichtsiederwäsche mit Phenol.

Die Zahlen an den Linien bezeichnen die folgenden Stoffströme:
- 1:: Phosgen
- 2:: Frisch-Katalysator
- 3:: Frisch-Monophenol
- 4:: Monophenol-Phosgen-Gemisch
- 5:: Chlorwasserstoff + Leichtsieder
- 6:: Reaktionsgemisch
- 7:: Chlorwasserstoff + Leichtsieder
- 8:: Reaktionsgemisch
- 9:: Chlorwasserstoff + Leichtsieder
- 10:: Reaktionsgemisch
- 11.: Reaktionsgemisch
- 12:: Chlorwasserstoff + Leichtsieder
- 13:: Reaktionsgemisch
- 14:: Monophenolkondensat
- 15:: Chlorwasserstoff
- 16:: Abfluss von Wasch-DPC
- 17:: Chlorwasserstoff
- 18:: Chlorwasserstoff
- 19:: Chlorwasserstoffendprodukt
- 20:: Sumpfprodukt der Chlorwasserstoffdestillation
- 21:: Leichtsiederbrüdenstrom
- 22:: Diarylcarbonat
- 23:: Diarylcarbonat
- 24:: Diarylcarbonat
- 25:: Leichtsiederbrüden
- 26:: Diarylcarbonat
- 27:: Diarylcarbonatendprodukt
- 28:: Diarylcarbonatsumpfprodukt
- 29:: Diarylcarbonatkondensat
- 30:: Katalysatorsalz in Diarylcarbonatsumpfprodukt
- 31:: Diarylcarbonat-Sumpfausschleusung
- 32:: Katalysatorlösungsrückführung.

### Beispiele:

### Bestimmung Salol-Gehalt

Salol (o-Hydroxy-Benzoesäurephenylester) wurde in den Diphenylcarbonat-Proben bei den Beispielen unter A) gaschromatographisch nach Hydrolyse des Titan-Salol-Komplexes mit Wasser vor der Destillation des Diphenylcarbonats bestimmt. Auf diese Weise kann die gesamte gebildete Menge des Nebenprodukts Salol im Diphenylcarbonat erfasst werden; andernfalls verbleibt ein Teil des gebildeten Salols an dem Titan komplex gebunden und entzieht sich damit der Erfassung als Nebenprodukt im Diphenylcarbonat nach dessen Destillation.

### YI

Die Bestimmung der optischen Eigenschaften der erfindungsgemäßen Formmassen geschieht durch Messung des sog. Yellowness-Index (YI) an Normprüfkörpern (60 x 40x 4 mm) nach ASTM E313. Die Herstellung der Normprüfkörper erfolgte bei einer Massetemperatur von 300 °C und einer Werkzeugtemperatur von 90 °C.

### A) Einfluss der Titan-Katalysatorrückführung auf die Bildung von Salol (o-OH-Bezoesäurephenylester) als Nebenprodukt in Diphenylcarbonat:

### Beispiel 1, nicht erfindungsgemäß

In einem 250ml Wellenbrechtopf mit Gaseinleitungsfritte (2 = 40-100 µm), Magnetrührer, Sumpf-Thermofühler, Intensivrückflußkühler, Septum für Probenahme und Ölheizbad mit Thermosteuerung wurden 141,2 g (1,5 Mol) Phenol und 355,7 mg (1,875 mMol) Titantetrachlorid (0,125 Mol% bez. auf Phenol) als Katalysator vorgelegt und auf 160°C aufgeheizt. Über einen Zeitraum von 2 Stunden wurden bei einer Sumpftemperatur von 160°C 88g (0,89 Mol) Phosgen (Molverhältnis: Phenol zu Phosgen wie 1,68 zu 1) über einen Massendurchflußmesser eingeleitet. Anschließend wurde die Gaseinleitungsfritte noch 2h mit N2-Gas gespült und die Sumpftemperatur von 160°C langsam auf 90°C abgesenkt. Danach wurde durch das Septum eine Probe für GC-Reinheitsbestimmung gezogen. Danach wurde mit wenig N2-Begasungnochmals 1h bei 90°C gerührt und dann eine 2. Kontrollprobe für die GC-Analyse gezogen. Danach wurde das Reaktionsgemisch im Vakuum von weniger als 30 mbar (a) und einer Sumpftemperatur von 210°C destilliert, wobei Leichtsieder und Diphenylcarbonat abdestilliert wurden. Das Katalysator-haltige Sumpfprodukt wurde für Folgeversuche wieder eingesetzt, um die Wiederverwendbarkeit dieser Katalysator-Zusammensetzung zu testen.

Die GC-Proben wurden vor der Messung bei RT gründlich mit Wasser geschüttelt, um Salol aus Komplexbindungen mit Titan frei zu setzen.

Salol-Gehalt bezogen auf DPC:0,4 Gew%.

### Beispiel 2, nicht erfindungsgemäß

Wie Beispiel 1. aber mit 177,8 mg (0.937 mMol) Titantetrachlorid (0,0625 Mol% bez. auf Phenol); Salol-Gehalt bezogen aufDPC:0,3 Gew%.

### Beispiel 3., 1. Katalysator-Rückführung

Wie Beispiel 1. aber mit Einsatz des Katalysator-haltigen Destillationssumpfes und nach Aufstockung des Katalysatorgehaltes mit frischen Titantetrachlorid auf einen Gesamtgehalt an Titan-Katalysator von 1,875 mMol (0,125 Mol% bez. auf Phenol);
Salol-Gehalt bezogen aufDPC:0,7 Gew%.

### Beispiel 4_{.} bis 7., 2. bis 5. Katalysator-Rückführung

Wie Beispiel 3. mit wiederholtem Einsatz des Katalysator-haltigen Destillationssumpfes und nach Aufstockung des Katalysatorgehaltes mit fi-ischen Titantetrachlorid auf einen Gesamtgehalt an Titan-Katalysator von 1,875 mMol (0,125 Mol% bez. auf Phenol);
Salol-Gehalt bezogen auf DPC bei 2. Katalysator-Rückführung: 1,0 Gew%.
Salol-Gehalt bezogen auf DPC bei 3. Katalysator-Rückführung: 1,2 Gew%.
Salol-Gehalt bezogen auf DPC bei 4. Katalysator-Rückführung: 1,2 Gew%.
Salol-Gehalt bezogen auf DPC bei 5. Katalysator-Rückführung: 1,1 Gew%.

### Beispiel 8., 1. Katalysator-Rückführung

Wie Beispiel 2. aber mit Einsatz des Katalysator-haltigen Destillationssumpfes und nach Aufstockung des Katalysatorgehaltes mit frischen Titantetrachlorid auf einen Gesamtgehalt an Titan-Katalysator von 0,937 mMol (0,0625 Mol% bez. auf Phenol);
Salol-Gehalt bezogen auf DPC:0,6 Gew%.

### Beispiel 9. bis 17., 2. bis 10. Katalysator-Rückführung

Wie Beispiel 8. mit wiederholtem Einsatz des Katalysator-haltigen Destillationssumpfes und nach Aufstockung des Katalysatorgehaltes mit frischen Titantetrachlorid auf einen Gesamtgehalt an Titan-Katalysator von 0,937 mMol (0,0625 Mol% bez. auf Phenol);
Salol-Gehalt bezogen auf DPC bei 2. Katalysator-Rückführung: 0,8 Gew%.
Salol-Gehalt bezogen auf DPC bei 3. Katalysator-Rückführung: 0,9 Gew%.
Salol-Gehalt bezogen auf DPC bei 4. Katalysator-Rückführung: 0,9 Gew%.
Salol-Gehalt bezogen auf DPC bei 5. Katalysator-Rückführung: 0,9 Gew%.
Salol-Gehalt bezogen auf DPC bei 6. Katalysator-Rückführung: 0,8 Gew%.
Salol-Gehalt bezogen auf DPC bei 7. Katalysator-Rückführung: 0,8 Gew%.
Salol-Gehalt bezogen auf DPC bei 8. Katalysator-Rückführung: 0,8 Gew%.
Salol-Gehalt bezogen auf DPC bei 9. Katalysator-Rückführung: 0,7 Gew%.
Salol-Gehalt bezogen auf DPC bei 10. Katalysator-Rückführung: 0,8 Gew%.

Die Daten sind nachfolgend tabellarisch (Tab. 1) zusammengefasst:

**Tabelle 1**

| Gew% Salol in DPC als Funktion der Titantetrachlorid-Menge in mol% bezogen auf Phenol und der Anzahl an Katalysator-Rückführungen | 0,125mol% | 0,0625mol% |
|---|---|---|
| 0. Katalysator-Rückführung | 0,4 | 0,3 |
| 1. Katalysator-Rückführung | 0,7 | 0,6 |
| 2. Katalysator-Rückführung | 1,0 | 0,8 |
| 3. Katalysator-Rückführung | 1,2 | 0,9 |
| 4. Katalysator-Rückführung | 1,2 | 0,9 |
| 5. Katalysator-Rückführung | 1,1 | 0,9 |
| 6. Katalysator-Rückführung | | 0,8 |
| 7. Katalysator-Rückführung | | 0,8 |
| 8. Katalysator-Rückführung | | 0,8 |
| 9. Katalysator-Rückführung | | 0,7 |
| 10. Katalysator-Rückführung | | 0,8 |

### B) Herstellung des Polycarbonats in der Schmelze

Aus einer Vorlage wurden 15927 kg/h Schmelzegemisch, bestehend aus 7798 kg DPC/h (36402mol/h) und 8128 kg einer BPA/Phenol-Mischung/h, unter Zusatz von 6,35 kg/h Katalysatorlösung durch einen Wärmetauscher gepumpt, auf 190 °C erwärmt und durch eine Verweilkolonne geführt. Die mittlere Verweilzeit betrug 45 min. Die Katalysatormischung bestand aus 0,52 kg des Phenoladduktes von Tetraphenylphosphoniumphenolat mit Phenol (enthalten 65,5 Gew.-% Tetraphenylphosphoniumphenolat 0,786 mol) gelöst in 4,5 kg Phenol. Die BPA/Phenolmischung bestand aus 92,66 Gew.-% BPA (32990 mol) und 7,34 Gew.-% Phenol (6339 mol). Das DPC/BPA-Verhältnis beträgt 1,103 (mol/mol).

Die Schmelze wurde dann über ein Entspannungsventil in einen unter 236 mbar stehenden Abscheider geleitet. Die abfließende Schmelze wurde in einen ebenfalls unter 236 mbar stehenden Fallfilmverdampfer wieder auf 190 °C erwärmt und in einer Vorlage aufgefangen. Nach einer mittleren Verweilzeit von 30 min wurde die Schmelze in die nächsten zwei gleichartig aufgebauten Stufen gepumpt. Bedingungen der 2./3. Stufe sind 95 / 50 mbar; 227 / 273 °C und 35 / 40 Minuten.

Das entstandene Oligomer wurde in einem sich anschließenden Korbreaktor (Reaktor 1) bei einem Druck von 7 mbar und einer Temperatur von 274 °C und einer mittleren Verweilzeit von 120 min weiter auskondensiert. Anschließend wurde die Schmelze in einem zweiten Korbreaktor (Reaktor 2) bei 290 °C und einem Druck von 0,8 mbar und einer mittleren Verweilzeit von 120 min weiter auskondensiert, aus dem Reaktor ausgetragen und granuliert.

Um den jeweiligen Einfluss des Salols auf die Farbzahl YI zu untersuchen, wurde entsprechend eine Salolmenge in das Schmelzegemisch dazugegeben.

Die Ergebnisse sind in Tab. 2 sowie in Fig. 1 zusammengefasst.

**Tabelle 2**

| Salol | Farbzahl |
|---|---|
| Gew.-% | |
| 0 | 0,37 |
| 0,01 | 0,4 |
| 0,05 | 0,42 |
| 0,1 | 0,44 |
| 0,5 | 0,48 |
| 1 | 0,63 |
| 2,5 | 1 |
| 5 | 1,5 |

### C) Verfahrensablauf zur Herstellung von gereinigtem Diphenylcarbonat:

Die Figuren erläutern den Ablauf des erfinderindungsgemässen Gesamtverfahrens am Beispiel der Herstellung von Diphenylcarbonat (DPC) aus Phenol und Phosgen unter Verwendung von Titantetrachlorid als Katalysator in einer besonders bevorzugten Ausführungsform ohne die Erfindung auf diesen Verfahrensablauf einzuschränken. Die Fig. 4 gibt einen Überblick über das Gesamtverfahren mit den Verfahrensabschnitten A) bis C), wie oben beschrieben.

Die Aufarbeitung des im Verfahrensabschnitt A), der Reaktion, hergestellten DPC erfolgt im Verfahrensabschnitt C), der mit Fig. 6 näher erläutert wird.

Das aus der letzten Reaktionsstufe F (Leichtsieder-Flashstufe) anfallende DPC-haltige Reaktionsgemisch wird als Stoffstrom 13 der Leichtsieder-Destillationskolonne K zugeleitet, in der Stoffe wie HCl, CO, CCl₄ und Phosgen nahezu vollständig entfernt und als Stoffstrom 21 der Kompressionsstufe Q und weiter als Strom 33 der HCl-Absorption R zugeleitet werden. Der in K von den Leichtsiedern befreite Stoffstrom 22 wird der Phenol-Flashstufe L zur weitegehenden Entfernung von Phenol zugeleitet, das als Stoffstrom 25 über die HCl-Absorption R wieder in die Reaktion zurückgeführt wird. Das so gereinigte DPC wird als Stoffstrom 23 der Phenol-Destillation M zugeführt, wo in einer Feindestillation die letzten Spuren an Leichtsiedern, wie insbesondere Phenol (als Strom 25) entfernt werden. Das so gereinigte DPC wird nun als Stoffstrom 24 der DPC-Destillation N zugeführt, in der das DPC destillativ von den Schwersiedern befreit wird und als hochreines Endprodukt 27 den Prozess verlässt. Die Schwersieder werden als Stoffstrom 28 dem DPC-Verdampfer O zugeführt, der die Aufgabe hat, die DPC-Verluste zu minimieren, und in dem zurückgewonnenes DPC als Stoffstrom 29 in die DPC-Destillationskolonne N zurückgeführt wird. Der verbleibende Sumpf im DPC-Verdampfer O kann entweder ganz oder teilweise in die Reaktion zurückgeführt werden, da er schwerflüchtige katalytisch aktive Titanester enthält, oder kann entsorgt werden.

Der Gehalt an Salol der oben genannten Stoffströme ist in der Tabelle 3 dargestellt.

**Tabelle 3**

| Strom | Salolgehalt (Gew.-%) |
|---|---|
| 13 | 2,02 |
| 22 | 2,06 |
| 23 | 2,36 |
| 24 | 0 |
| 25 | 0,06 |
| 27 | 0 |
| 28 | 0 |
| 29 | 0 |
| 30 | 0 |

## Patentansprüche

1. Verfahren zur Herstellung von Diarylcarbonat durch Umsetzung von Monohydroxyaromaten mit Phosgen in der Schmelze in Gegenwart von einer oder mehrerer Lewis-Säuren als Katalysator(en) und ohne Zugabe von zusätzlichem Lösemittel, **dadurch gekennzeichnet, dass** der oder die Katalysator(en) zu 50 Gew% oder mehr aus einer Aufarbeitungsstufe des Verfahrens in die Reaktionsstufe des Verfahrens zurückgeführt wird (werden).

2. Verfahren nach Anspruch 1.), **dadurch gekennzeichnet, dass** der Katalysator in der Aufarbeitung als Sumpfprodukt, welches weitere hochsiedende Bestandteile enthalten kann, abgetrennt wird und die Reaktionsstufe des Verfahren zurückgeführt wird.

3. Verfahren nach Anspruch 1.) oder 2.), **dadurch gekennzeichnet, dass** das Verfahren kontinuierlich erfolgt.

4. Verfahren nach Anspruch 1.), **dadurch gekennzeichnet, dass** der Katalysator Titantetrachlorid, Titantetraphenolat, oder Aluminiumtrichlorid, oder Derivate dieser Verbindungen ist.

5. Verfahren nach Anspruch 1.), 2.) oder 3.), **dadurch gekennzeichnet, dass** der Katalysator oder eine Mischung aus wenigstens zwei Katalysatoren aus der Gruppe der Verbindungen von Titan, Zirkon und Aluminium ausgewählt ist.

6. Verfahren nach einem der Ansprüche 1.) bis 5.), **dadurch gekennzeichnet, dass** die Umsetzung von Monohydroxyaromaten mit Phosgen in Gegenwart von wenigstens einem Katalysator bei Drücken von 8 bis 20 bar und Temperaturen von 120°C bis 230°C durchgeführt werden.

7. Verfahren nach einem der Ansprüche 1.) bis 6.), **dadurch gekennzeichnet, dass** der im Verfahren gebildete Chlorwasserstoff wenigstens einer Reinigungsmaßnahme, ausgewählt aus der Gruppe von Kondensation, Wäsche oder Destillation unterworfen wird.

## Claims

1. Process for preparing diaryl carbonate by reacting monohydroxyaromatics with phosgene in the melt in the presence of one or more Lewis acid(s) as catalyst(s) and without addition of additional solvent, **characterized in that** 50% by weight or more of the catalyst(s) is/are recycled from a workup stage of the process into the reaction stage of the process.

2. Process according to Claim 1.), **characterized in that** the catalyst is removed in the workup as the bottom product which may comprise further high-boiling constituents and the reaction stage of the process is recycled.

3. Process according to Claim 1.) or 2.), **characterized in that** the process is effected continuously.

4. Process according to Claim 1.), **characterized in that** the catalyst is titanium tetrachloride, titanium tetraphenoxide or aluminium trichloride, or derivatives of these compounds.

5. Process according to Claim 1.), 2.) or 3.), **characterized in that** the catalyst or a mixture of at least two catalysts is selected from the group of the compounds of titanium, zirconium and aluminium.

6. Process according to any of Claims 1.) to 5.), **characterized in that** the reaction of monohydroxyaromatics with phosgene is performed in the presence of at least one catalyst at pressures of 8 to 20 bar and temperatures of 120°C to 230°C.

7. Process according to any of Claims 1.) to 6.), **characterized in that** the hydrogen chloride formed in the process is subjected to at least one purification measure selected from the group of condensation, scrubbing and distillation.

## Revendications

1. Procédé de fabrication de carbonate de diaryle par mise en réaction de composés monohydroxyaromatiques avec du phosgène dans la masse fondue en présence d'un ou de plusieurs acides de Lewis en tant que catalyseurs et sans ajout d'un solvant supplémentaire, **caractérisé en ce que** le ou les catalyseurs sont recyclés à 50 % en poids ou plus depuis une étape de traitement du procédé dans l'étape de réaction du procédé.

2. Procédé selon la revendication 1, **caractérisé en ce que** le catalyseur est séparé lors du traitement en tant que produit de fond, qui peut contenir d'autres constituants de point d'ébullition élevé, et recyclé dans l'étape de réaction du procédé.

3. Procédé selon la revendication 1 ou 2, **caractérisé en ce que** le procédé a lieu en continu.

4. Procédé selon la revendication 1, **caractérisé en ce que** le catalyseur est le tétrachlorure de titane, le tétraphénolate de titane ou le trichlorure d'aluminium, ou des dérivés de ces composés.

5. Procédé selon la revendication 1, 2 ou 3, **caractérisé en ce que** le catalyseur ou un mélange d'au moins deux catalyseurs est choisi dans le groupe des composés de titane, de zirconium et d'aluminium.

6. Procédé selon l'une quelconque des revendications 1 à 5, **caractérisé en ce que** la mise en réaction de composés monohydroxyaromatiques avec du phosgène est réalisée en présence d'au moins un catalyseur à des pressions de 8 à 20 bar et des températures de 120 °C à 230 °C.

7. Procédé selon l'une quelconque des revendications 1 à 6, **caractérisé en ce que** le chlorure d'hydrogène formé dans le procédé est soumis à au moins une mesure de purification, choisie dans le groupe constitué par une condensation, un lavage ou une distillation.
